# EUROPÄISCHE PATENTSCHRIFT

(11) **EP 1 364 651 B1**
(45) Veröffentlichungstag und Bekanntmachung des Hinweises auf die Patenterteilung: **02.11.2005**
(21) Anmeldenummer: 03011450.8
(22) Anmeldetag: 20.05.2003
(51) Int. Cl.: A61K 33/06, A61K 9/06, A61K 9/10, A61K 9/107, A61P 17/00, A61P 17/16

(54) **Pharmazeutische Zubereitung mit hautentquellender Wirkung**
Pharmaceutical composition with deswelling properties
Composition pharmaceutique dégonflant la peau

(30) Priorität: 24.05.2002 DE 10223222
(43) Veröffentlichungstag der Anmeldung: 26.11.2003
(73) Patentinhaber: Sebapharma GmbH & Co. KG, 56154 Boppard-Bad Salzig (DE)
(72) Erfinder: Gottfreund, Joachim, Dr., 56154 Boppard (DE); Meyer, Thomas, Dipl.-Ing., 56281 Emmelshausen (DE)
(74) Vertreter: Lippert, Stachow & Partner

(56) Entgegenhaltungen:
- EP-A- 0 925 783
- GLOOR, M. ET AL.: "Action of an Aluminium Chlorohydrate and Glycerol Containing Skin Protection Cream in Experimental Skin Irritation Produced by Sodium Laurylsulfate and Solvents" DERMATOLOGIE IN BERUF UND UMWELT, Bd. 49, Nr. 1A, 2001, Seiten 67-70, XP001153953 Germany
- PERRENOUD, D. ET AL.: "Efficacy of a Protectve Cream in a Real-world Apprentice Hairdresser Environment" DERMATOLOGIE IN BERUF UND UMWELT, Bd. 49, Nr. 1A, 2001, Seiten 71-72, XP001153955 Germany
- PERRENOUD, D. ET AL.: "Effect of a Protective Cream against Skin Irritation Associated with Hand Hygiene Practices" DERMATOLOGIE IN BERUF UND UMWELT, Bd. 49, Nr. 1A, 2001, Seiten 91-94, XP001153954 Germany
- BOCK, M. ET AL.: "Okklusionseffekt von Schutzhandschuhen" DERMATOLOGIE IN BERUF UND UMWELT, Bd. 49, Nr. 1A, 2001, Seiten 85-87, XP002250084 Germany

## Beschreibung

Die vorliegende Erfindung betrifft die Verwendung einer Zusammensetzung mit hautentquellender Wirkung, zur Herstellung eines topisches Präparats zur Prophylaxe und/oder Behandlung von Hautekzemen, -rötungen, -trockenheit, -einrissen, -schuppungen, -juckreiz und -quellung, die Aluminiumsalze umfasst.

Eine intakte Homschicht, d. h. eine epidermale Barriere, ist eine wesentliche Voraussetzung für die Schutzfunktion der Haut gegenüber äußeren Einflüssen. Der länger dauernde oder ständig wiederholte Kontakt mit Wasser führt zur Schädigung der epidermalen Barriere und der darunter liegenden Hautschichten [1], d. h. der lebenden Epidermis. Die gleiche Situation, verbunden mit einem Feuchtigkeits- und Wärmestau, stellt sich ein, wenn über einen längeren Zeitraum durch Latex-Handschuhe u. ä. oder Chemikalien ein Gasaustausch zwischen Haut und Umgebung unterbunden wird, die Hornschicht quillt, was als Mazeration der Haut bezeichnet wird [2]. Durch die Beeinträchtigung der Barrierefunktion kommt es zur stärkeren Einwirkung von äußeren Stoffen auf die Haut und dem Verlust körpereigener Stoffe wie Wasser und/oder Elektrolyte aus der Haut, d. h. es führt zu einem transepidermalen Wasserverlust [3]. Es entsteht ein Abnutzungsekzem, das als klinisches Bild Rötung, Trockenheit, Schuppung, Einrisse, Juckreiz der Haut zeigt, und das bei mangelndem Hautschutz in ein chronisches Ekzem übergehen kann.

Darüber hinaus können potentielle Allergene leicht in die Haut eindringen und zur Sensibilisierung führen [4]. Die Entstehung von allergischen Kontaktekzemen kann durch das Abnutzungsekzem gefördert werden.

Für dieses Problem werden z. Zt. Präparate angeboten, die die geschädigte Barriere wieder herstellen sollen oder die Haut vor dem Kontakt mit externen Noxen schützen [5]. Für verschiedene Berufsgruppen wie Friseure u. ä. sind daher bienenwachshaltige oder fluorkohlenwasserstoffhaltige Hautschutzpräparate empfohlen worden [6]. Die Wirkung dieser Präparate besteht darin, dass sie eine chemische oder physiologische Barriere auf der Haut herstellen, die verhindert, dass Wasser oder andere Irritationen an sie gelangen. Sie haben aber keine entquellende Wirkung.

Auch durch Emulsionen, die Öl-in-Wasser oder Wasser-in-Öl-Emulsionen darstellen, werden nur die in Defizit geratenen Inhaltsstoffe der Haut wieder aufgefüllt, z. B. hautfeuchtigkeitssteigernde und fettende Verbindungen. Dabei können in O/W-Systemen sogar durch die eingesetzten Emulgatoren zusätzliche desikierende Wirkungen erzeugt werden [7].

Diese kosmetisch akzeptablen Präparationen sind daher wirkungsloser als W/O-Lotionen oder - Cremes, wobei diese wiederum den Nachteil haben, dass sie auf gequollener Haut nicht applizierbar sind, da sich auf der Haut kein zusammenliegender Fettfilm ausbilden kann. Durch derartige Präparate wird die Hornschicht nicht in den ursprünglichen Zustand zurückgeführt, d. h. entgequollen.

Weiterhin werden in kosmetischen Präparaten hauptsächlich als desodorierende und adstringierende Wirkstoffe Aluminiumsalze in einer Konzentration zwischen 10 bis 25 Gew.-% eingesetzt. Diese in Wasser gelösten Salze zeichnen sich durch eine erhöhte irritative Wirkung auf die Haut aus [9]. Mit "Adstringentien" werden Stoffe bezeichnet, "die durch Reaktion mit dem Eiweiß oberster Gewebeschichten zur Verdichtung des kolloidalen Gefüges mit Bildung einer fest zusammenhängenden oberflächlichen Membran führen". Zu dieser Gruppe gehören auch Tannin und andere Gerbstoffe [10].

Aus der US 4,781,917, US 5,776,494 und US 5,549,887 sind beispielsweise Aluminiumchlorohydrat und Natriumaluminiumchlorohydratlactat als schweißhemmend wirkende Aluminiumsalze bekannt. Die schweißhemmenden Wirkstoffe werden dabei vorzugsweise auf der Basis eines Gels als Träger eingesetzt und finden als Deodoranten Verwendung. Basische Aluminiumchloride bzw. aktivierte Aluminiumchlorohydrate sind im US-Patent Nr. 3,904,741 oder in EP 0 183 171 A1 beschrieben. Solche basischen Aluminiumchloride sind unter dem Markennamen REACH® im Handel erhältlich.

EP 0 925 783 A1 beschreibt Hautschutzzubereitungen mit aktiviertem Aluminiumchlorohydrat als Grundstoff, welcher meist geringe Mengen verschiedener Feuchthaltemittel zugesetzt werden, darunter auch Natriumlactat.

Bisher gibt es kein Produkt, dass Haut durch Entquellung der gequollenen Haut in ihren Ausgangszustand wieder zurückführt.

Eine entquellend wirkende Präparation zeichnet sich dadurch aus, dass der transepidermale Wasserverlust der Haut gesenkt und die Hautrauhigkeit reduziert wird. Wünschenswert ist, dass nach Applikation des Produktes die Hautfeuchtigkeit den Ausgangswert nach möglichst kurzer Zeit wieder erreicht [8].

Es besteht eine Nachfrage nach einer Zusammensetzung, die gequollene Haut durch Entquellung in ihren Ausgangszustand zurückführt. Eine derartige entquellend wirkende Zusammensetzung muss bewirken, dass der transepidermale Wasserverlust der Haut gesenkt und gleichzeitig die Rauhigkeit der Haut reduziert wird, sodass nach Applikation der Zusammensetzung nach einer möglichst kurzen Zeitdauer die Hautfeuchtigkeit ihren Ausgangszustand erreicht.

Die Aufgabe der vorliegenden Erfindung ist es daher, eine Zusammensetzung bereitzustellen, die entquellend auf Haut wirkt.

Die Aufgabe wird erfindungsgemäß durch die Verwendung einer Zusammensetzung mit hautentquellender Wirkung zur Herstellung eines topischen Präparats zur Prophylaxe und/oder Behandlung Hautekzemen, -rötungen, -trockenheit, -einrissen, -schuppungen, juckreiz und - quellung gelöst, wobei die Zusammensetzung Aluminiumchlorohydrat und mindestens ein dreiwertiges Aluminiumsalz mit einem organischen Liganden enthält, der ausgewählt ist aus Aluminiumchlorohydratlactat und/oder Aluminiumchlorohydratcitrat und/oder Aluminiumchlorohydratmalat, einschließlich ihrer Alkali- und Erdalkalisalze und als Grundlage einen pharmazeutisch akzeptablen Träger und gegebenenfalls ein oder mehrere übliche kosmetische und/oder pharmazeutische Hilfs-, Verarbeitungs- und/oder Zusatzstoffe enthält, wobei die Zusammensetzung einen pH-Wert von zwischen 4,0 bis 7,0 aufweist.

In einer bevorzugten Ausführungsform der vorliegenden Erfindung wird Natriumaluminiumchlorohydroxylactat (festes Chloracel) verwendet.

In einer bevorzugten Ausführungsform ist Aluminiumchlorohydrat in einer Konzentration von 5 bis 30 Gew.-% und das Aluminiumsalz in einer Konzentration von 2 bis 10 Gew.-% in der Zusammensetzung enthalten.

Die Zusammensetzung umfasst neben dem Wirkstoff einen Träger, der wenigstens ein flüssiges Basismaterial, wenigstens einen pH-Regulator, und gegebenenfalls wenigstens eine Ölkomponente umfasst, die gegebenenfalls die Wirkung des Wirkstoffs verstärken können bzw. dem Benutzer ein angenehmes Hautgefühl bei dem Anwenden der Zusammensetzung verschaffen.

In einer bevorzugten Ausführungsform umfasst der Träger ein flüssiges Basismaterial, das Wasser und/oder Alkohole mit 2 bis 4 Kohlenstoffatomen und/oder deren Mischungen umfasst. In bevorzugten Ausführungsformen ist das flüssige Basismaterial Ethanol, das aus Kostengründen, beispielsweise mit 1 % Diethylphthalat, vergällt sein kann, oder Isopropanol, welche die Trocknungszeit von feucht formulierten Produkten beschleunigen und einen angenehmen kühlenden Effekt bewirken. Weiterhin können Butanol, n-Propanol, sec-Butanol und Isobutanol verwendet werden. Es können auch Mischungen der vorstehenden Alkohole eingesetzt werden, sowie Mischungen aus einem oder mehreren Alkoholen mit Wasser. In dem letzteren Fall liegt das Gewichtsverhältnis von Alkohol zu Wasser vorzugsweise zwischen 1:5 und 4:5.

Zum Einstellen eines optimalen pH-Werts umfasst der Träger der erfindungsgemäßen Zusammensetzung wenigstens einen pH-Regulator, der ausgewählt ist aus der Gruppe, bestehend aus Milchsäure oder einem Salz davon, Zitronensäure oder einem Salz davon oder deren Mischungen, wobei die Salze insbesondere Alkali- und Erdalkalisalze umfassen, oder Alkali wie beispielsweise Natronlauge. Die Zusammensetzung weist einen pH-Wert auf, der im Bereich von leicht sauer bis neutral liegt, d. h. von 4,0 bis 7,0, da die vorstehenden Aluminiumverbindungen im alkalischen Milieu hydrolysieren können. Vorzugsweise ist der pH-Wert der erfindungsgemäßen Zusammensetzung 5,5. Der Anteil des zugesetzten pH-Regulators liegt vorzugsweise im Bereich von 5 bis 20 Gew.-%. Er ist besonders bevorzugt 15 Gew.-%.

In weiteren bevorzugten Ausführungsformen umfasst der Träger der Zusammensetzung gemäß der Erfindung weiterhin wenigstens eine Ölkomponente. Die erfindungsgemäß verwendete Ölkomponente kann aus mehreren Stoffklassen ausgewählt werden. Die Ölkomponente umfasst beispielsweise Ester aus gesättigten und/oder ungesättigten, verzweigten und/oder unverzweigten Alkoholen einer Kettenlänge von 3 bis 30 Kohlenstoffatomen und gesättigten und/oder ungesättigten, verzweigten und/oder unverzweigten Alkancarbonsäuren einer Kettenlänge von 3 bis 30 Kohlenstoffatomen und/oder aromatischen Carbonsäuren. Bevorzugte Esteröle umfassen Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleylerucat, Erucyloleat, Erucylerucat sowie synthetische, halbsynthetische und natürliche Gemische solcher Ester wie z. B. Jojobaöl.

Weiterhin können als Ölkomponente Kohlenwasserstoffe oder Kohlenwasserstoffwachse, verwendet werden. Als Beispiele seien Paraffinöl, Squalan und Squalen genannt.

Weiterhin eignen sich gesättigte und/oder ungesättigte, verzweigte und/oder unverzweigte Alkohole, beispielsweise Octyldodecanol, oder Dialkylether, beispielsweise Dicaprylylether, als Ölkomponente.

In einer bevorzugten Ausführungsform werden Fettsäuretriglyceride als Ölkomponente verwendet, namentlich Triglycerinester gesättigter und/oder ungesättigter, verzweigter und/oder unverzweigter Alkancarbonsäuren einer Kettenlänge von 8 bis 24, vorzugsweise 12 bis 18 Kohlenstoffatomen. Die Ölkomponente kann hierbei ein synthetisches, halbsynthetisches oder natürliches Öl, beispielsweise Olivenöl, Sonnenblumenöl, Sojaöl, Erdnussöl, Rapsöl, Mandelöl, Palmöl, Kokosöl, Nerzöl oder Rizinusöl und dergleichen, sein.

Weiterhin kommen im Sinne der vorliegenden Erfindung Silikonöle als Ölkomponente in Betracht, die vorzugsweise linear oder cyclisch sind. Die verwendeten Silikonöle sind vorzugsweise Dialkyl- und Alkylarylsiloxane. In bevorzugten Ausführungsformen wird als Silikonöl Octamethylcyclotetrasiloxan, Hexamethylcyclotrisiloxan, Polydimethylsiloxan und Poly(methylphenylsiloxan) verwendet. Octamethylcyclotetrasiloxan wird besonders bevorzugt verwendet. Vorzugsweise umfasst eine Ölkomponente, die ein Silikonöl oder eine Mischung aus Silikonölen enthält, einen zusätzlichen Gehalt an einer weiteren Ölkomponente.

Selbstverständlich können erfindungsgemäß nämlich auch Mischungen der vorstehend genannten Öle, Fette und Wachse bzw. Stoffe und Verbindungen als Ölkomponente der vorliegenden Erfindung eingesetzt werden.

Bei Verwendung von wenigstens einer Ölkomponente liegt der Ölkomponenten-Anteil im Bereich von 0,1 bis 10 Gew.-%, basierend auf der Gesamtzusammensetzung.

In bevorzugten Ausführungsformen besteht die Ölkomponente gemäß der vorliegenden Erfindung aus Mischungen aus C₁₂₋₁₅- Alkylbenzoat und 2-Ethylhexylisostearat, Mischungen aus C₁₂₋₁₅-Alkylbenzoat und Isotridecylisononanoat oder Mischungen aus C₁₂₋₁₅-Alkylbenzoat, 2-Ethylhexylisostearat und Isotridecylisononanoat.

In weiteren bevorzugten Ausführungsformen wird als Ölkomponente eine Mischung aus Octamethylcyclotetrasiloxan und Isotridecylisononanoat oder eine Mischung aus Octamethylcyclotetrasiloxan und 2-Ethylhexylisostearat verwendet.

In einer besonders bevorzugten Ausführungsform besteht die verwendete Ölkomponente ausschließlich aus dem Wachs Cetylpalmitat.

Weiterhin werden vorzugsweise 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäuretriglycerid, Dicaprylylether und deren Mischungen als Ölkomponente verwendet.

Gegebenenfalls ist mindestens ein Emulgator, Coemulgator, Stabilisator, Dispergator, Verdickungsmittel, Feuchthaltemittel, Konservierungsstoff, Duftstoff, Chemikalie, Geruchsabsorber, Pigment, Farbstoff, desodorierender Wirkstoff, filmbildender Stoff, Lichtschutzmittel, Antioxidans, Enzym, Weichmacher, fungistatisches Mittel, bakteriostatisches Mittel und/oder Pflegestoff als kosmetische und/oder pharmazeutische Hilfs-, Verarbeitungs-und/oder Zusatzstoffe in der Zusammensetzung enthalten, deren Gehalt zwischen 0 bis 20 Gew.-% liegt, basierend auf der Gesamtzusammensetzung.

Erfindungsgemäß werden insbesondere nicht-ionische Emulgatoren verwendet, die im Allgemeinen eine bessere Hautverträglichkeit als anionische Emulgatoren aufweisen. Vorzugsweise werden Emulgatoren verwendet, die Anlagerungsprodukte von 10 bis 50 Mol-% Ethylenoxid an Fettalkohole, Fettsäuren, Fettsäurealkanolamide, Fettsäuremonoglyceride, Sorbitanfettsäureester, Methylglycosidfettsäureester oder Polyglycolether-modifizierte Polysiloxane sind.

Der Zusatz eines Coemulgators ist vorteilhaft zur Ausbildung von sehr feinteiligen Dispersionen und weist im Allgemeinen einen lipophilen Charakter auf. Erfindungsgemäß verwendete Coemulgatoren umfassen gesättigte Alkohole mit 8 bis 24 Kohlenstoffatomen, ethoxylierte oder propoxylierte Alkohole und Carbonsäuren mit 8 bis 24 Kohlenstoffatomen, Ester aus einem Polyol und einer Fettsäure, Mono- und Polyglycerinester, Mono- und Polyglycerinether, Propylenglycolester, Methylglucoseester, Polyglycerinmethylglucoseester und deren Mischungen.

Erfindungsgemäß eingesetzte Verdickungsmittel umfassen Hydroxyethylcellulose, Hydroxypropylcellulose, Polyacrylsäure, Polyvinylpyrrolidon, Polydimethylsiloxan, Polymethylphenylsiloxan, Polyacrylat, Dextran, Siliciumdioxid und Aluminiumsilikat. In einer bevorzugten Ausführungsform der vorliegenden Erfindung ist das verwendete Verdickungsmittel bei ölig-alkoholischen Gelen Siliciumdioxid oder ein Aluminiumsilikat und bei wässerig-alkoholischen oder alkoholischen Gelen ein Polyacrylat.

Um die Feuchtkeitsregulierung der Haut weiterhin zu unterstützen, kann die erfindungsgemäße Zusammensetzung weiterhin Feuchthaltemittel umfassen. Erfindungsgemäß werden insbesondere 1,2-Propandiol, 1,4-Butandiol, Polyole wie Glycerin, Diglycerin, Triglycerin, 1,2,6-Hexantriol, Ethylenglycol, Propylenglycol, Butylenglycol, Erythrit, Polyethylenglycol-4 (PEG-4), PEG-6, PEG-7, PEG-8, PEG-9, PEG-10, PEG-12, PEG-14, PEG-16, PEG-18, PEG-20, Sorbeth-6, Sorbeth-20, Sorbeth-30, Sorbeth-40, Fructose, Glucose, Maltose, Sorbit, Sucrose, Maltitol, Inosit, Xylose, Xylit, Glucoronsäure, Hyaluronsäure, Pyrrolidoncarbonsäure und deren Mischungen verwendet.

Beispiele der erfindungsgemäß verwendeten Konservierungsstoffe umfassen Benzoesäure und ihre Derivate, wie beispielsweise Ammonium-, Natrium-, Kalium-, Magnesium-, Propyl-, Butyl-und Phenylbenoatbenzoat, Salicylsäure, Phenol, Benzylalkohol, 4-Hydroxybenzoesäure, 4-Hydroxybenzoesäureester, Triclosan, Halocarban, Hydantoin, Harnstoff, Sorbinsäure Dehydroessigsäure, Isoxazol, Oxazol, quarternäre Ammoniumverbindungen, Formaldehyd, Glutaraldehyd, Glyoxal, Hexamidin, Isopropylkresol, Peptid-Antibiotika, die Salze und/oder Derivate der vorstehenden Verbindungen und deren Mischungen.

Jeder Duftstoff kann verwendet werden, soweit er üblicherweise in Zusammensetzungen verwendet wird.

Im Sinne der vorliegenden Erfindung umfassen die eingesetzten Chemikalien Vitamin A, Vitamin B₂, Vitamin E, Magnesiumascorbinsäure-2-phosphat, Dikaliumglycyrrhizinat, Glutathion und Weizenkeimöl.

Erfindungsgemäß verwendete Geruchsabsorber sind beispielsweise Zeolithe, Zinkricinoleat, Cyclodextrine, Metalloxide, Chlorophyll und Silikate. Als Metalloxid wird vorzugsweise Aluminiumoxid verwendet. Vorzugsweise verwendete Silikate sind ausgewählt aus der Gruppe der Schichtsilikate, insbesondere Kaolinit, Montmorillonit, Saponit, Hectorit, Bentonit, Talkum, Ilit, Smectit, Beidelit und Nontronit.

Als Pigmente werden erfindungsgemäß Titandioxid, Eisenoxid, Zinkoxid und Kaolin verwendet, um die kosmetische Akzeptanz des Präparats beim Anwender zu unterstützen.

Erfindungsgemäß werden unter desodorierenden Wirkstoffen Stoffe verstanden, die als Geruchsüberdecker, Geruchsabsorber und keimhemmende Mittel wirken. Keimhemmende Mittel umfassen beispielsweise Organohalogenide.

Als Antioxidantien können Aminosäuren, α-Hydroxysäuren, Peptide, Imidazol, Carotine, Carotinoide, Thioverbindungen, Sulfoximinverbindungen, Metallchelatoren, Folsäure, Bilirubin, Catechine, Flavonoide, Tocopherol, Gallussäureester, Hydrochinon, Ascorbinsäure, Isoascorbinsäure, Tocopherole, Zimtsäure, Butylhydroxytoluol, Butylhydroxyanisol, Harnsäure, Mannose, Zinkverbindungen, Selenverbindungen, deren Derivate oder Salze der vorstehenden Verbindungen verwendet werden. Weiterhin können Antioxidantien-haltige Pflanzenextrakte eingesetzt werden.

Als Beispiele für Weichmacher können Di-n-butylphthalat, Diethylsebacat, Diisopropyladipat, Ethylethylcarbomethylphthalat, Tetradecan, Propylenethylenglycolmonolaurat, ethoxyliertes oder propxyliertes Butanol, Hexylenglycol und Ethyl-1,3-hexandiol genannt werden.

Beispiele für filmbildende Stoffe sind Aloe vera und Konjac-Manane.

Weiterhin können der Zusammensetzung dem Fachmann geläufige Lichtschutzmittel, Stabilisatoren, Dispergatoren, Farbstoffe, Enzyme, fungistatische Mittel, bakteriostatische Mittel, keimtötende Mittel und Pflegestoffe zugesetzt werden.

Eine besonders bevorzugte Ausführungsform der vorliegenden Erfindung umfasst eine Zusammensetzung, die 5-30 Gew.-% Aluminiumchlorohydrat und 2 bis 15 Gew.-% Aluminiumchlorohydratlactat und/oder -citrat, Glycerin, Sorbitol, Propylenglycol, Ethoxydiglycol, Aloe vera, Konjac-Manane, Hyaluronsäure, Squalan und einen pH-Wert zwischen 4,0 bis 7,0 aufweist.

Die erfindungsgemäße Zusammensetzung, d.h. insbesondere die pharmazeutische Zusammensetzung, kann in Form von Gel, Lipogel, Creme, Salbe, Lotion, Schaum, mit und ohne Treibmittel oder Druckgas vorliegen. Im Sinne der Erfindung schließt dies Emulsionen, Oleogele, Hydrodispersionen und Lipodispersionen ein.

Als Treibmittel oder Druckgas können erfindungsgemäß entweder ein verflüssigbares Gas oder ein Permanentgas oder eine Mischung der vorstehenden verwendet werden. Beispiele für verflüssigbare gasförmige Treibmittel sind Fluorkohlenwasserstoffe und deren Mischungen und Kohlenwasserstofftreibmittel und deren Mischungen, wie beispielsweise Butan, Isobutan, Propan oder Isopentan. Als Permanentgase sind Kohlendioxid, Stickstoff oder Distickstoffmonoxid geeignet. Die Menge des verwendeten Treibmittels wird dabei durch die üblichen, auf dem Aerosolgebiet bekannten Faktoren bestimmt. Weiterhin kann die Zusammensetzung gemäß der Erfindung auch in Form eines Sprays ohne Verwendung eines Treibmittel abgegeben werden, wenn sie in einem mit einer fingerbetriebenen Pumpe versehenen Behälter enthalten ist.

Das hergestellte topische Präparat zur Prophylaxe und/oder Behandlung von Hautekzemen, - rötungen, -trockenheit, -einrissen, -schuppungen, -juckreiz und -quellung gemäß der vorliegenden Erfindung ist nicht auf die Anwendung im Humanbereich beschränkt, sondern vielmehr bei allen Säugetieren anwendbar.

Folgende Beispiele und Figuren sollen die Erfindung erläutern, ohne sie jedoch einzuschränken.
Figur 1 zeigt die Wirkung der Behandlung mit erfindungsgemäßen und nicht erfindungsgemäßen Zusammensetzungen nach Okklusion durch Beurteilung mittels Episkopie im Vergleich zu Kontrollen.
Fig. 2 zeigt die Wirkung der Behandlung mit erfindungsgemäßen und nicht erfindungsgemäßen Zusammensetzungen nach Okklusion durch Beurteilung mittels eines Corneometers im Vergleich zu Kontrollen.
Fig. 3 zeigt die Wirkung der Behandlung mit erfindungsgemäßen und nicht erfindungsgemäßen Zusammensetzungen nach Okklusion durch Beurteilung mittels eines Tewameters im Vergleich zu Kontrollen.
Figur 4 zeigt eine erste Kammerpenetrationen der Kontrolle.
Figur 5 zeigt eine erste Kammerpenetrationen unter Verwendung von NaOH.
Figur 6 zeigt eine erste Kammerpenetration einer nicht erfindungsgemäßen Zusammensetzung.
Figur 7 zeigt erste Kammerpenetration einer erfindungsgemäßen Zusammensetzung.
Figuren 8, 9, 10 bzw. 11 zeigen eine zweite Kammerpenetration der Kontrolle, unter Verwendung von NaOH, einer nicht erfindungsgemäßen Zusammensetzung bzw. erfindungsgemäßen Zusammensetzung.
Figuren 12, 13, 14 bzw. 15 zeigen eine dritte Kammerpenetration der Kontrolle, unter Verwendung von NaOH, einer nicht erfindungsgemäßen Zusammensetzung bzw. erfindungsgemäßen Zusammensetzung.
Figuren 16, 17, 18 bzw. 19 zeigen eine vierte Kammerpenetration der Kontrolle, unter Verwendung von NaOH, einer nicht erfindungsgemäßen Zusammensetzung bzw. erfindungsgemäßen Zusammensetzung.
Figuren 20, 21, 22 bzw. 23 zeigen eine fünfte Kammerpenetration der Kontrolle, unter Verwendung von NaOH, einer nicht erfindungsgemäßen Zusammensetzung bzw. erfindungsgemäßen Zusammensetzung.

Die Erfindung wird nun durch Beispiele erläutert, die jedoch nur veranschaulichend sind und den Schutzumfang der Erfindung nicht einschränken.

| **Beispiel 1** | | |
|---|---|---|
| Xanthan Gum | 1 Gew.-% | |
| Milchsäure | 1,6 Gew.-% | |
| Sorbit | 3 Gew.-% | |
| Propylenglycol | 3 Gew.-% | |
| Glycerin | 3 Gew.-% | |
| Chloracel fest | 4,4 Gew.-% | |
| Na-Lactat | 15 Gew.-% | |
| Locron L | 21 Gew.-% | |
| | | Parfümöle, Wasser ad 100 Gew.-% pH-Wert 5,5 |

| **Beispiel 2** | | |
|---|---|---|
| PEG-40 hydriertes | | |
| Rizinusöl | 2 Gew.-% | |
| Glycerin | 2 Gew.-% | |
| Chloracel fest | 4,4 Gew.-% | |
| Locron L | 21 Gew.-% | |
| Milchsäure | 1,6 Gew.-% | |
| Na-Lactat | 15 Gew.-% | Parfümöle, Wasser ad 100 Gew.-% pH-Wert 5,5 |

| **Beispiel 3** | | |
|---|---|---|
| Chloracel fest | 4,4 Gew.-% | |
| Locron L | 21 Gew.-% | |
| Milchsäure | 1,6 Gew.-% | |
| Na-Lactat | 15 Gew.-% | |
| Hydroxydecylcellulose | 1 Gew.-% | |
| Parfüm | 1 Gew.-% | Parfümöle, Wasser ad 100 Gew.-% pH-Wert 5,5 |

| **Beispiel 4** | | |
|---|---|---|
| Octyldodecanol | 2 Gew.-% | |
| C₁₂₋₁₅ Alkylbenzoat | 2 Gew.-% | |
| C₁₀₋₃₀Alkylacrylat | 0,15 Gew.-% | |
| Locron L | 21 Gew.-% | |
| Chloracel fest | 4,4 Gew.-% | |
| Milchsäure | 1,6 Gew.-% | |
| Na-Lactat | 15 Gew.-% | Parfümöle, Wasser ad 100 Gew.-% pH-Wert 5,5 |

| **Beispiel 5** | | |
|---|---|---|
| Locron LIC F1. | 21 Gew.-% | |
| Lactolin pH 5,6 | 15 Gew.-% | |
| Natrural Extract AP | 5 Gew.-% | |
| Chloracel fest | 4,4 Gew.-% | |
| Dehyquart A-CA | 3 Gew.-% | |
| Purac 80% | 2,1 Gew.-% | |
| Merquat 281 | 2 Gew.-% | Parfümöle, Wasser ad 100 Gew.-% pH-Wert 5,5 |

| **Beispiel 6** | | |
|---|---|---|
| Locron L | 21 Gew.-% | |
| Butylen Glycol | 5 Gew.-% | |
| Crodamol PMP | 5 Gew.-% | |
| Emulgade CM | 5 Gew.-% | |
| Natural Extract AP | 5 Gew.-% | |
| Chloracel fest | 4,4 Gew.-% | |
| Natronlauge 45% | 1 Gew.-% | |
| Structure Solanace | 0,9 Gew.-% | |
| Zitronensäure-Monohydrat | 0,8 Gew.-% | |
| Natrosol 250 HBR | 0,45 Gew.-% | Parfümöle, Wasser ad 100 Gew.-% pH-Wert 5,5 |

| **Vergleichsbeispiel 1 (VB1)** | | |
|---|---|---|
| Fitoderm | 8 Gew.-% | |
| Emulgade CM | 5 Gew.-% | |
| Konjac Mannane 1,0 MEP | 2,5 Gew.-% | |
| Natrosol 250 HHBR | 0,5 Gew.-% | |
| Natriumchlorid | 10 Gew.-% | |
| Natronlauge 45% | 0,2 Gew.-% | Parfumöle, Wasser ad 100 Gew.-% pH-Wert 5,5 |

| **Vergleichsbeispiel 2 (VB2)** | | |
|---|---|---|
| Natriumchlorid | 10 Gew.-% | |
| FinsolvTN | 3,25 Gew.-% | |
| MC 30 | 3,25 Gew.-% | |
| Montanov 68 | 3 Gew.-% | |
| Bienenwachs, weiß | 1,5 Gew.-% | |
| Lactolin pH 5,6 | 1 Gew.-% | |
| Keltrol TF | 0,3 Gew.-% | |
| Natronlauge 45% | 0,1 Gew.-% 1 | Parfümöle, WasserAqua ad 100 pH-Wert 5,5 |

Die vorstehenden Ausgangsstoffe haben folgende INCI-Namen und können von dem angegebenen Hersteller bezogen werden.

| | |
|---|---|
| 1,3-Butandiol | INCI: Butylenglycol (96%ige Lösung), Hersteller: Merck |
| Chloracel fest | INCI: Natriumaluminiumchlorhydroxylactat, Hersteller: Interorgana |
| Locron L | INCI: Aluminiumchlorohydrat (50%ige Lösung), Hersteller: Höchst AG |
| Locron LIC Fl | INCI: Aluminiumchlorohydrat (50%ige Lösung), Hersteller: Clariant |
| Dehyquart A-CA | INCI: Cetrimoniumchlorid (30%ige Lösung), Hersteller: Cognis |
| Purac | INCI: Natriumlactat (80%ige Lösung), Hersteller: Purac Biochem |
| Merquat 281 | INCI: Polyquatemium-22 (42,5%ige Lösung), Hersteller: Chemviron |
| Crodamol PMP | INCI: PPG-2-Myristyletherpropionat, Hersteller: Croda GmbH |
| Emulgade CM | INCI: Cetearylisononanoat (und) Ceterareth-20 (und) Cetearylalkohol (und) Glycerylstearat (und) Glycerin (und) Cetylplamitat (und) Ceteareth-12, Hersteller: Henkel |
| Structure Solanace | INCI: Modifizierte Kartoffelstärke (86%ige Lösung), Hersteller: S. Black |
| Natrosol 250 HBR | INCI: Hydroxyethylcellulose, Hersteller: Hercules |
| Natural Extract AP | INCI: Betain, Hersteller: Rovi GmbH |
| Fitoderm | INCI: Squalan, Hersteller: Cognis |
| Konjac Mannane | INCI: Mannan, Hersteller: GfN |
| Natrosol 250 HHBR | INCI: Hydroxyethylcellulose, Hersteller: Hercules |
| FinsolvTN | INCI: C12-C15 Alkylbenzoat, Hersteller: Erbslöh |
| MC 30 | INCI: hydriertes Polyisobuten, Hersteller: N&R |
| Montanov 68 | INCI: Cetearylalkohol und Cetearylglucosid, Hersteller: Interorgana |
| Bienenwachs, weiß | INCI: Cera alba, Hersteller: Kahl & Co. |
| Lactolin | INCI: Natriumlactat (50%ige Lösung), Hersteller: Boehringer |
| Keltrol TF | INCI: Xanthan Gum (94%ige Lösung). Hersteller: Biesterfeld |

Um die Wirksamkeit der vorliegenden Zusammensetzungen darzulegen, wurde der Einfluss der zwei erfindungsgemäßer Zusammensetzungen gemäß Beispiel 5 (B5) und Beispiel 6 (B6) auf experimentell gequollene Hornschicht *in vivo* und *in vitro* untersucht. Die Untersuchungen wurden unter Verwendung von dem Fachmann bekannten Geräten, Messmethoden und Messbedingungen durchgeführt, wenn nicht anders angegeben.

### Untersuchung zum Einfluss zweier erfindungsgemäßer Zusammensetzungen auf experimentell gequollene Hornschicht in vivo

Das methodische Vorgehen simulierte eine Situation, wie sie beim prolongierten Tragen okklusiv wirkender Operationshandschuhe nach vorheriger chirurgischer Handwäsche gegeben ist. Dazu wurde entsprechend des Vorgehens beim repetitiven Waschtest die Unterarmbeugeseite von 15 freiwilligen Probanden mit NLS gewaschen (Gehring et al, Predictive Washing Test for Evaluation the Individual Eczema Risk. Contact Dermatitis 39, 8-13. 1998). Im Anschluss an die Waschung wurde das gesamte Testfeld mit einer Folie für 24 Stunden okklusiv abgedeckt. Unmittelbar nach der Okklusionsphase wurden die zwei vorstehenden Zusammensetzungen standardisiert appliziert und deren Soforteffekt nach 5 Minuten beurteilt.

Die Okklusion führt aufgrund eines Quelleffektes zu einer Vergröberung des Hautoberflächenreliefs und zu einer ausgeprägteren Hautrauhigkeit. Damit ist ein Anstieg des transepidermalen Wasserverlustes verbunden, der die okklusiv bedingte Irritation widerspiegelt. Unmittelbar nach Okklusion geht der erhöhte transepidermale Wasserverlust in die Kapazitätsmessung mit ein, so dass sich erhöhte Werte darstellen. Bereits 5 Minuten später wird allerdings - ebenfalls als Zeichen der Irritation - ein Verlust an Hornschichtfeuchtigkeit ersichtlich.

Die Hautrauhigkeit wurde episkopisch mit dem Visiometer beurteilt (Ruhr J.W., Gehring W., Gloor M.: Analyse der Hautrauhigkeit bei Personen unterschiedlicher Altersgruppen mit dem Visiometer - Ein neuartiges EDV-gestütztes Messverfahren. Akt. Derm. 21, 151-156, 1995). Bei diesem Verfahren drückt eine Abnahme des Messwertes eine Hautglättung und eine Verminderung der Hautrauhigkeit aus.

Die Untersuchungen erfolgten an den Vorlarseiten beider Unterarme. Die Zusammensetzung gemäß Beispiel 5 wurde zusammen mit einem unbehandelten Kontrollfeld (Kontrolle 1, K1) zu einer Gruppe zusammengefasst und an einem Unterarm getestet. Kontralateral erfolgte die Untersuchung der Zusammensetzung gemäß Beispiel 6 im Vergleich zu einem unbehandelten Kontrollfeld (Kontrolle 2, K2). Alle Messwerte sind Differenzen zum Ausgangswert nach Okklusion (AW). Die statistische Auswertung erfolgte für jeden Testblock getrennt nach dem Wilcoxon Paardifferenzentest für verbundene Stichproben.

Die Untersuchungen erfolgten an einem Kollektiv von 15 gesunden Probanden. Davon waren 11 weiblich mit einem Durchschnittsalter von 36,5 Jahren. Die Altersgrenzen lagen bei 20 und 60 Jahren. Die 4 männlichen Probanden hatten ein Durchschnittsalter von 32,5 Jahren. Hier waren Altersgrenzen von 19 und 48 Jahren gegeben.

### Die Ergebnisse sind wie folgt:

### Episkopie

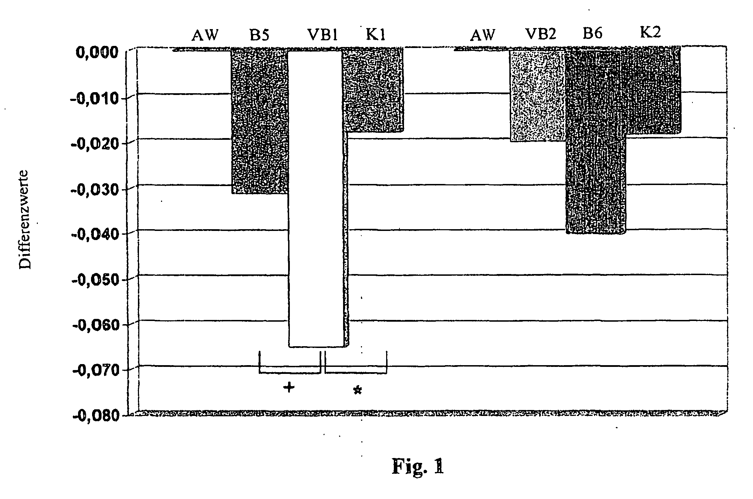

Nach Beendigung der Okklusion kommt es auch unbehandelt zu einer Reduktion der Hautrauhigkeit, die allerdings durch Anwendung von der Zusammensetzung gemäß Beispiel 5 bzw. Beispiel 6 verbessert wird. Insbesondere hat die Zusammensetzung gemäß Beispiel 6 einen befriedigenden glättenden Effekt erkennen lassen. Die Ergebnisse sind in Fig. 1 dargestellt. Fig. 1 zeigt die Wirkung der erfindungsgemäßen Zusammensetzungen nach Okklusion durch Beurteilung mittels Episkopie.

### Transepidermaler Wasserverlust

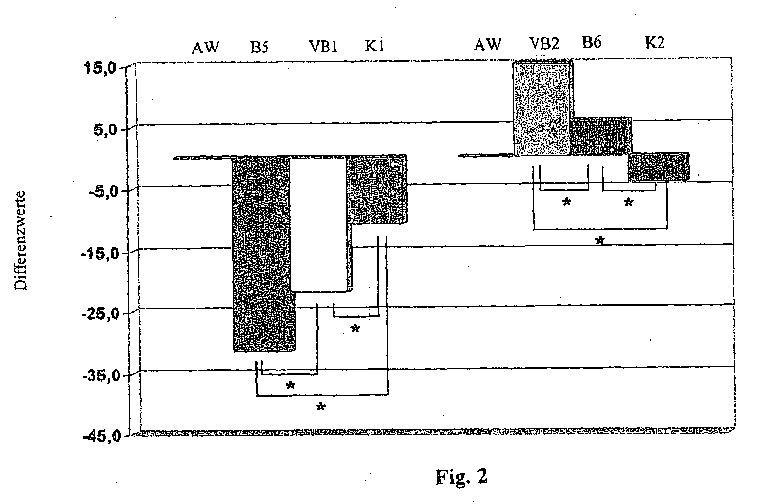

Die Zusammensetzungen gemäß Beispiel 5 bzw. Beispiel 6 haben eine bessere Reduktion des irritativ bedingten Anstiegs des TEWL herbeigeführt als dies bei der unbehandelten Kontrolle der Fall gewesen ist. Die Ergebnisse sind in Fig. 2 dargestellt. Fig. 2 zeigt die Wirkung der erfindungsgemäßen Zusammensetzungen nach Okklusion durch Beurteilung mittels eines Corneometers.

### Hornschichtfeuchtigkeit

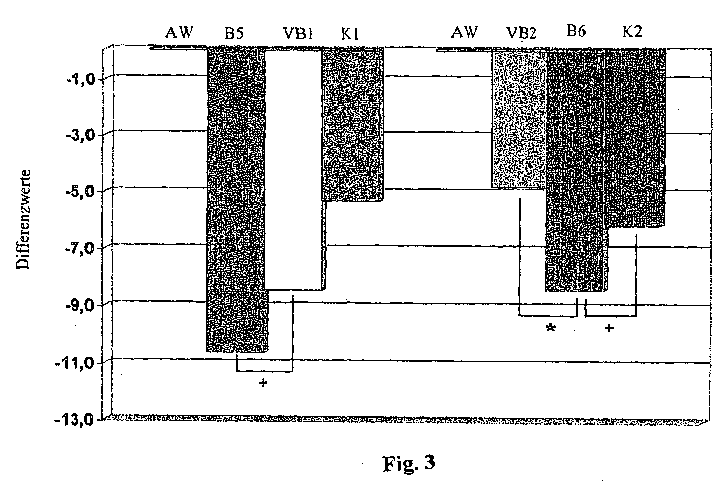

Insbesondere die Zusammensetzung gemäß Beispiel 6 hat den durch die Okklusion bedingten Verlust an Hornschichtfeuchtigkeit vollständig verhindert und im Sinn einer Positivbilanz gegenüber zum unbehandelten Kontrollfeld zu einer statistisch signifikanten Verbesserung der Hornschichtfeuchtigkeit geführt. Die Ergebnisse sind in Fig. 3 dargestellt. Fig. 3 zeigt die Wirkung der erfindungsgemäßen Zusammensetzungen nach Okklusion durch Beurteilung mittels eines Tewameters.

Prolongierte Okklusion in Kombination mit intensiver Hautreinigung durch NLS führt zu einer Zunahme der Hautrauhigkeit, zu einem erhöhten transepidermalen Wasserverlust und zu einer Reduktion der Homschichtfeuchtigkeit. Eine strikte Korrelation des Effektes der zwei verschiedenen Zusammensetzung bei der Beeinflussung der unterschiedlichen Bewertungskriterien (Hautrauhigkeit, TEWL, Homschichtfeuchtigkeit) ist nicht gegeben. Bei kritischer Abwägung aller Kriterien ist insbesondere die Zusammensetzung gemäß Beispiel 6 besonders zur Entquellung der Haut geeignet. Bei der Zusammensetzung gemäß Beispiel 6 kommt zum glättenden Effekt ein hydratisierender Einfluss hinzu.

### Untersuchungen zum Einfluss zweier erfindungsgemäßer Zusammensetzungen auf experimentell gequollene Hornschicht in vitro

Bei einer vorausgegangenen Untersuchung wurden zwei Zusammensetzungen gemäß Beispiel 5 bzw. Beispiel 6 auf ihren hautglättenden Einfluss bei experimentell durch Okklusion gequollener Hornschicht untersucht. Dabei ließ die Zusammensetzung gemäß Beispiel 6 episkopisch betrachtet die größte Abnahme der Hautrauhigkeit erkennen. Ziel der weiterführenden Untersuchung war es, den direkten Einfluss auf das gequollenen Stratum corneum zu visualisieren.

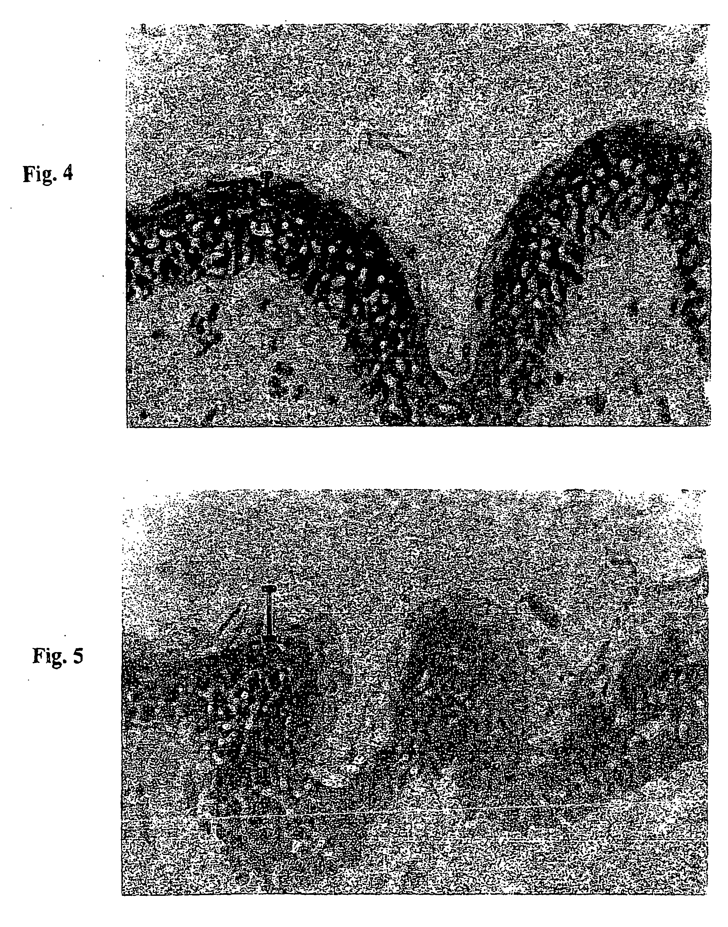

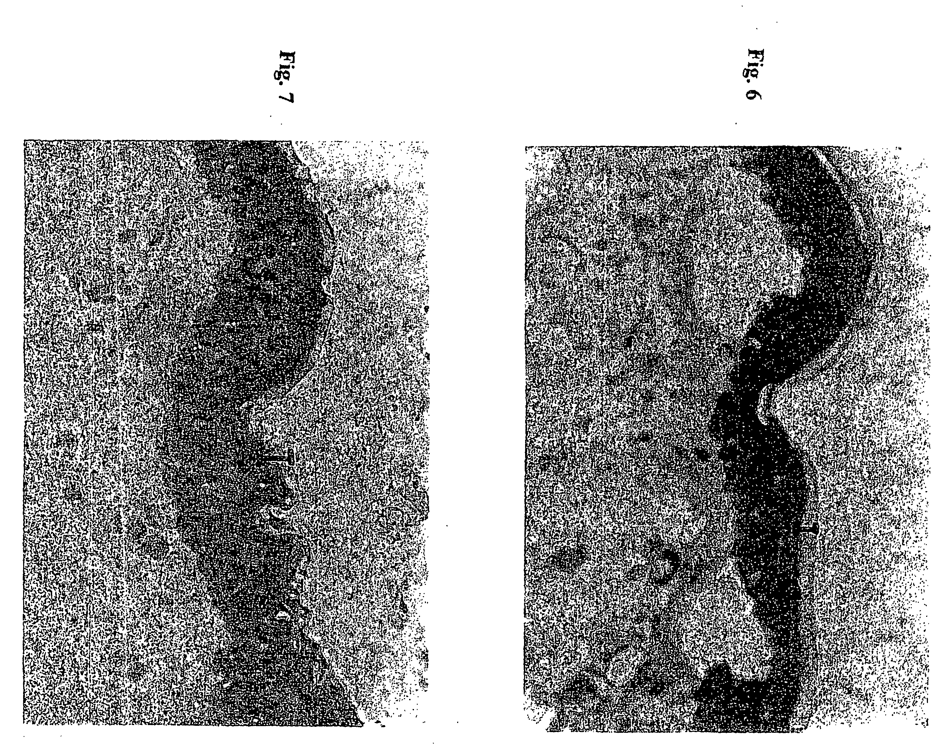

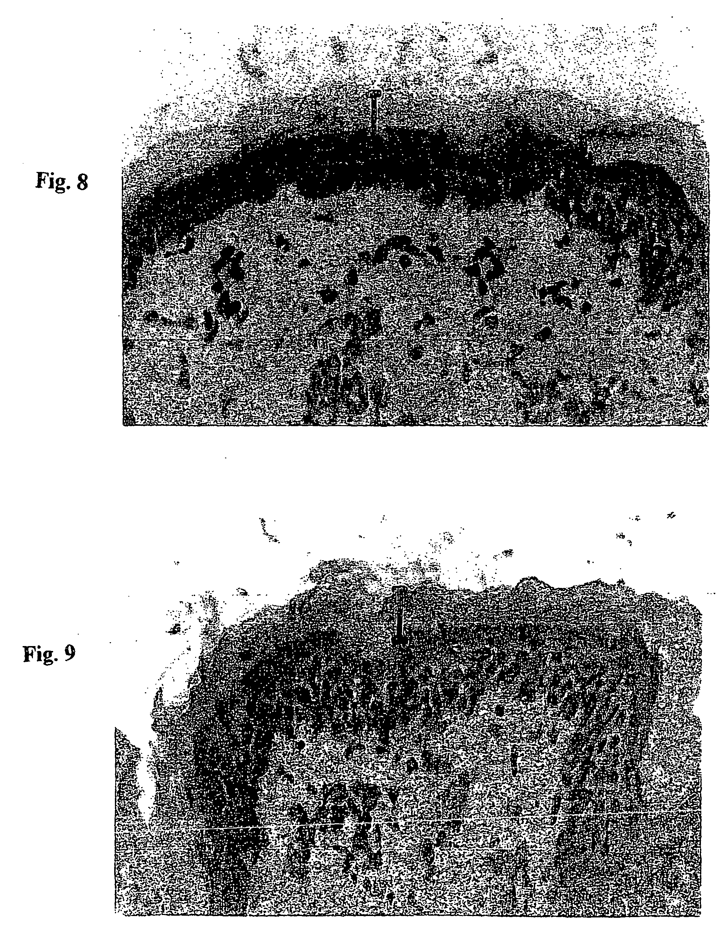

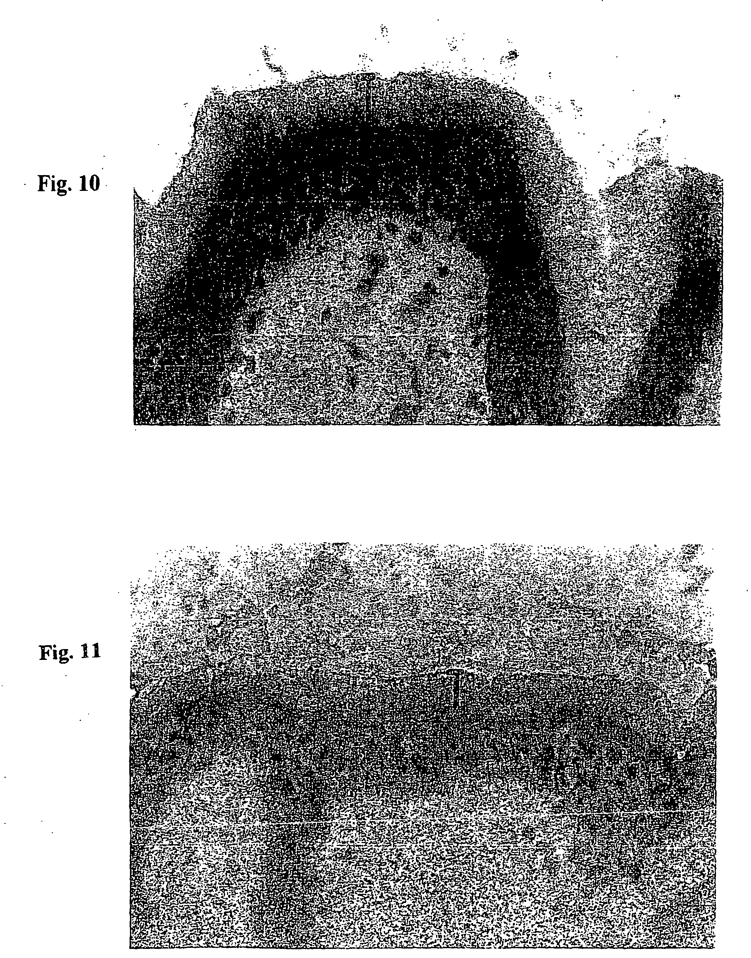

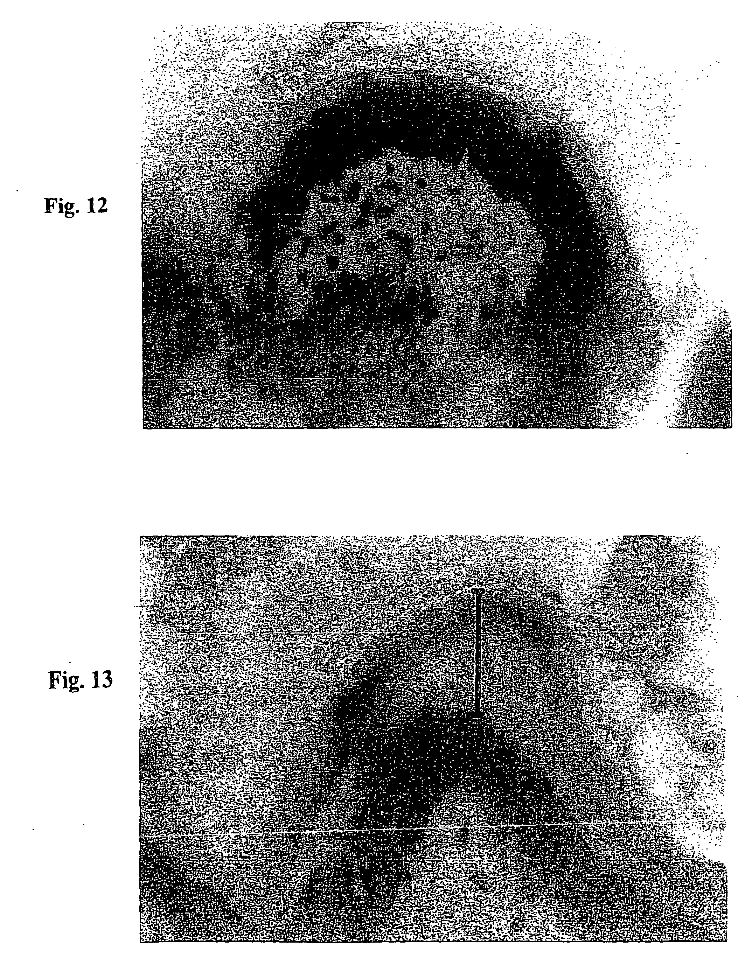

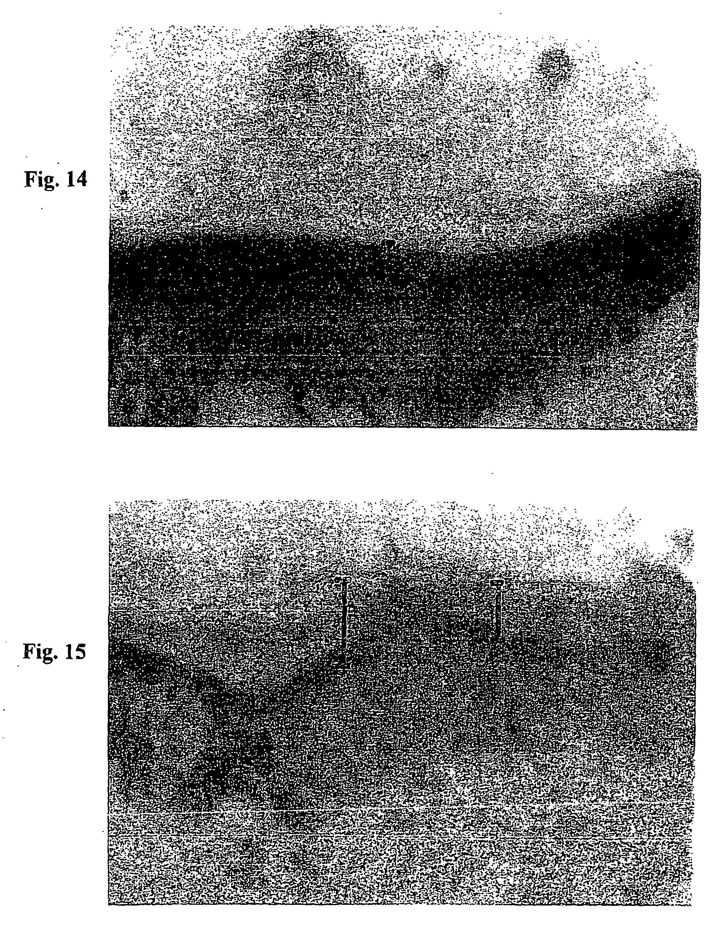

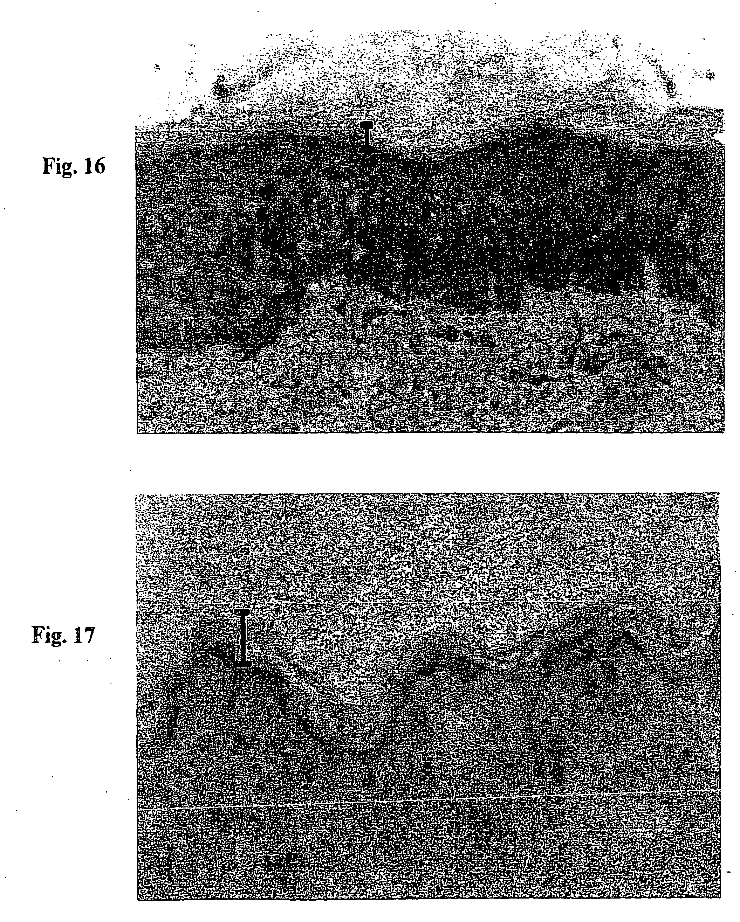

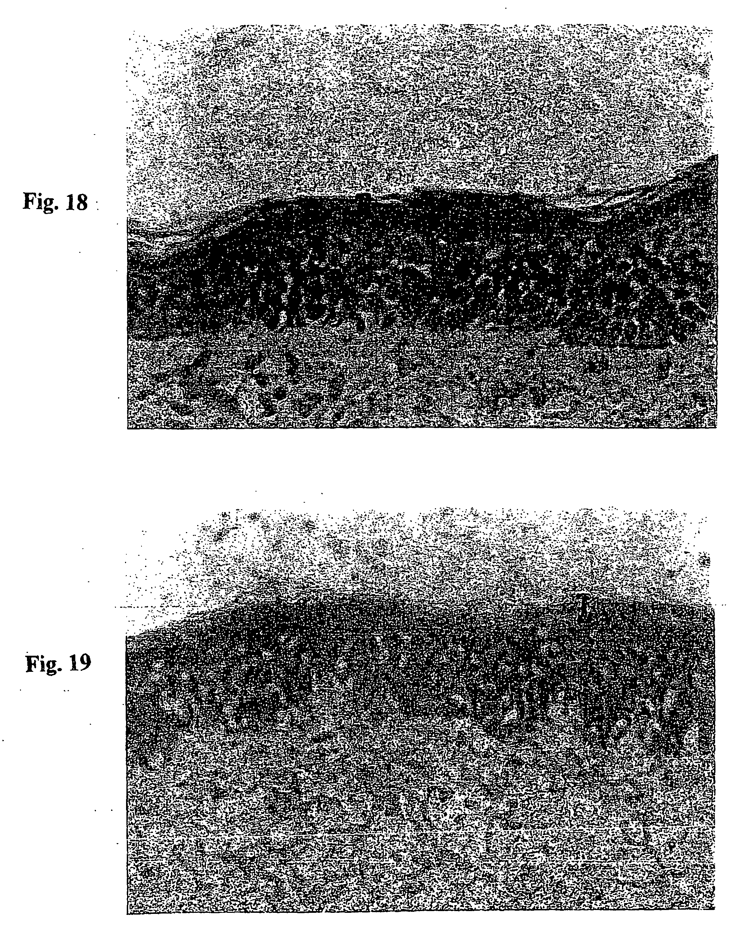

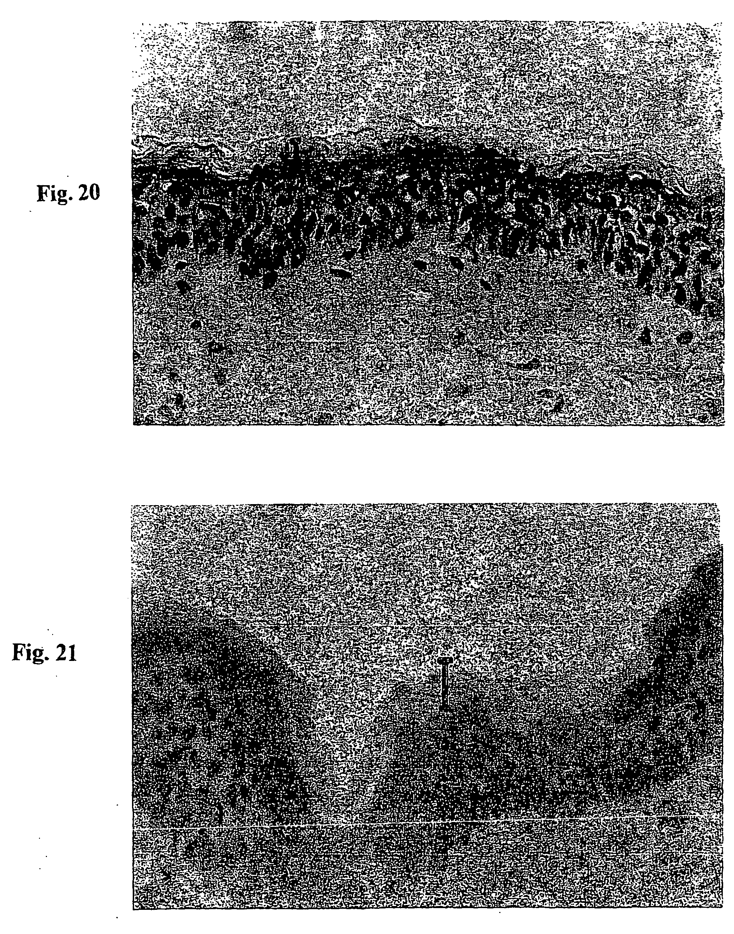

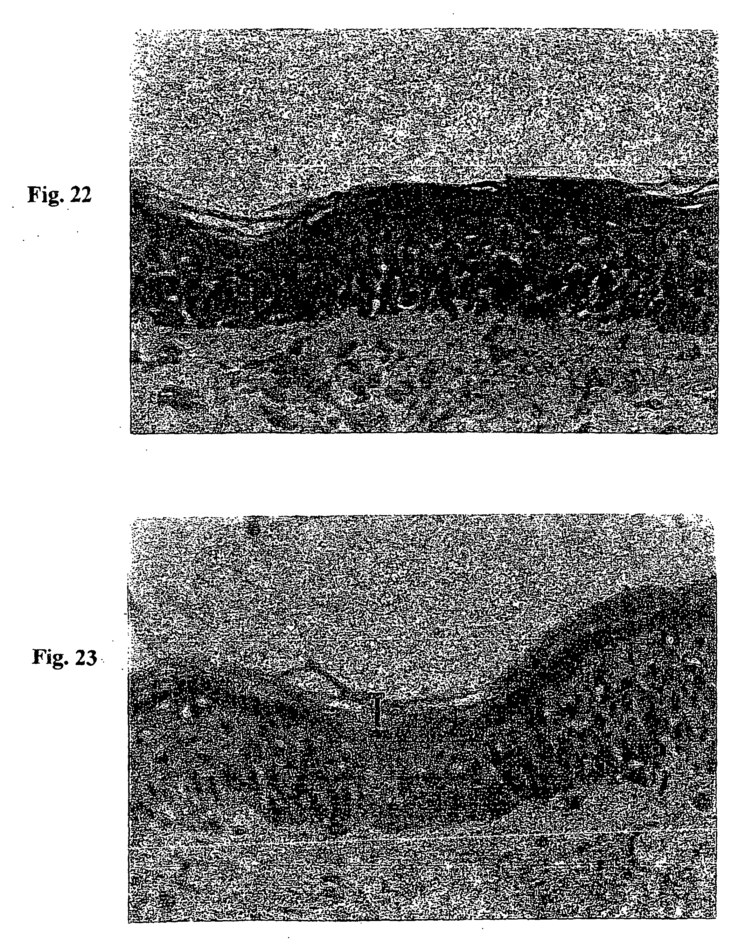

Bei der Zusammensetzung gemäß Beispiel 5 handelt es sich um eine klare, transparente Formulierung, die am Gefrierschnittmodell beurteilt werden konnten, wobei in diesem Fall 10 Gefrierschnittmodelle beurteilt wurden. Für die Zusammensetzung cremiger, nicht transparenter Konsistenz - wie dies bei der Zusammensetzung gemäß Beispiel 6 der Fall ist - verbietet sich dieses Verfahren. Hier wurde eine modifizierte Kammerpenetration in zwei Schritten vorgenommen. Zuerst erfolgte eine Inkubation mit NaOH, um eine Homschichtquellung zu provozieren. Nach 5 Minuten Inkubationszeit wurden Überstände von NaOH abpippetiert. Nach anschließender Spülung mit NaCl erfolgte die Behandlung mit der Zusammensetzung gemäß Beispiel 6. Abschließend wurde unbehandelte, mit NaOH gequollene und mit der Zusammensetzung inkubierte Haut mikroskopisch untersucht. Die histologischen Präparate wurden nach HE gefärbt. Die Ergebnisse der fünf durchgeführten Kammerpenetrationen der Zusammensetzung gemäß Beispiel 6, gemäß Vergleichsbeispiel 2 sowie der Kontrolle und mit Natronlauge sind in Figuren 4 bis 23 dargestellt. Bei Gewebe Nr. 2 ist die unbehandelte Hornschicht schon erheblich gequollen.

Bei der Untersuchung am Kryostatschnitt hat die Zusammensetzung gemäß Beispiel 5 bei allen 10 untersuchten Gewebeproben übereinstimmend eine hervorragende entquellende Wirkung auf das experimentell gequollene Stratum corneum erkennen lassen. Bei der Kammerpenetration hat die Zusammensetzung gemäß Beispiel 6 ebenfalls gute Resultate gezeigt.

Mit beiden Vorgehensweisen, d. h. Gefrierschnitt und Kammerpenetration, ist die morphologische Beurteilung der entquellenden Wirkung an der Hornschicht möglich gewesen. Die Ergebnisse sind eindeutig und gut reproduzierbar und zeigen deutliche die Wirkung der erfindungsgemäßen Zusammensetzungen.

### LITERATUR:

[1]
   Hornstein: "Berufsbedingte allergische Dermatosen", TW Dermatologie 25, 110 - 121, 1995
[2]
   Göring, Kröning, Ziemer: "Kontaktallergien bei medizinischem Personal", Der Deutsche Dermatologe 7, 727 - 733, 1993
[3]
   Fluhr, Lazzerini, DIstante, Gloor, Berardesca: "Effects of Prolonged Occlusion on Stratum corneum Barrier Function and Water Holding Capacity", Skin Pharmacol Appl Skin Physiol 1999, 12: 193 - 198
[4]
   Odia, Pürschel, Vocks, Rakoski: "Noxen und Irritantien im Beruf", Dermatosen 42, Heft 5, 179-183, 1994
[5]
   Schwanitz: "Prävention chronischer Handekzeme", Der Deutsche Dermatologe 9, 931 - 938, 1993
[6]
   Pilz, Frosch: "Hautschutz für Friseure", Dermatosen 42, Heft 5, 199 - 202, 1994
[7]
   Gehring: "Desikierende Wirkung von Emulgatoren" (Nachweis über Autor)
[8]
   Gehring: "Untersuchung zum Einfluss verschiedener Prüfpräparate auf die experimentell gequollene Hornschicht in vivo", 2002 (unveröffentlicht)
[9]
   Aluminiumtrichlorid-6-Wasser: Dermatika, Niedner, Ziegenmeyer, S. 318
[10]
   Adstringentien: Pschyrembel Klinisches Wörterbuch, 255. Aufl., S.23

## Patentansprüche

1. Verwendung einer Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, juckreiz und -quellung, wobei die Zusammensetzung Aluminiumchlorohydrat und mindestens ein dreiwertiges Aluminiumsalz mit einem organischen Liganden, ausgewählt aus Aluminiumchlorohydratlactat und/oder Aluminiumchlorohydratcitrat und/oder Aluminiumchlorohydratmalat, einschließlich ihrer Alkali- und Erdalkalisalze und als Grundlage einen pharmazeutisch akzeptablen Träger und gegebenenfalls ein oder mehrere übliche kosmetische und/oder pharmazeutische Hilfs-, Verarbeitungs- und/oder Zusatzstoffe enthält, wobei die Zusammensetzung einen pH-Wert von zwischen 4,0 bis 7,0 aufweist.

2. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 1, **dadurch gekennzeichnet, dass** die Zusammensetzung Aluminiumchlorohydrat in einer Konzentration von 5 bis 30 Gew.-% und das Aluminiumsalz in einer Konzentration von 2 bis 10 Gew.-% enthält.

3. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, juckreiz und -quellung gemäß Anspruch 1 oder 2, **dadurch gekennzeichnet, dass** der pH-Wert 5,5 ist.

4. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß irgendeinem der vorstehenden Ansprüche 1 bis 3, **dadurch gekennzeichnet, dass** der Träger wenigstens ein flüssiges Basismaterial, wenigstens einen pH-Regulator, und gegebenenfalls wenigstens eine Ölkomponente umfasst.

5. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 4, **dadurch gekennzeichnet, dass** das flüssige Basismaterial Wasser, Alkohole mit 2 bis 4 Kohlenstoffatomen oder deren Mischungen umfasst.

6. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 4, **dadurch gekennzeichnet, dass** der pH-Regulator Milchsäure, Zitronensäure, Milchsäuresalze, Zitronensäuresalze oder deren Mischungen, wobei die Salze insbesondere Alkali- und Erdalkalisalze umfassen, oder Alkali umfasst.

7. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 4, **dadurch gekennzeichnet, dass** die Ölkomponente Ester aus gesättigten und/oder ungesättigten, verzweigten und/oder unverzweigten Alkoholen einer Kettenlänge von 3 bis 30 Kohlenstoffatomen und gesättigten und/oder ungesättigten, verzweigten und/oder unverzweigten Alkancarbonsäuren einer Kettenlänge von 3 bis 30 Kohlenstoffatomen und/oder aromatischen Carbonsäuren, Kohlenwasserstoffe, Kohlenwasserstoffwachse, Silikonöle, Dialkylether, gesättigte und/oder ungesättigte, verzweigte und/oder unverzweigte Alkohole und Fettsäuretriglyceride und deren Mischungen umfasst.

8. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -Juckreiz und -quellung gemäß Anspruch 7, **dadurch gekennzeichnet, dass** die Ölkomponente ausgewählt ist aus der Gruppe der Esteröle, bestehend aus Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleylerucat, Erucyloleat, Erucylerucat sowie synthetische, halbsynthetische und natürliche Gemische solcher Ester, aus der Gruppe der Triglycerinester gesättigter und/oder ungesättigter, verzweigter und/oder unverzweigter Alkancarbonsäuren einer Kettenlänge von 8 bis 24, vorzugsweise 12 bis 18 Kohlenstoffatomen, aus der Gruppe der synthetischen, halbsynthetischen und natürlichen Ölen, bestehend aus Olivenöl, Sonnenblumenöl, Sojaöl, Erdnussöl, Rapsöl, Mandelöl, Palmöl, Kokosöl, Jojobaöl, Nerzöl und Rizinusöl, aus der Gruppe der Kohlenwasserstoffe und -wachse, bestehend aus Paraffinöl, Squalan und Squalen, aus der Gruppe der Silikonöle, bestehend aus Octamethylcyclotetrasiloxan, Hexamethylcyclotrisiloxan, Polydimethylsiloxan, Poly(methylphenylsiloxan) und deren Mischungen.

9. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 7, **dadurch gekennzeichnet, dass** die Ölkomponente Cetylpalmitat ist.

10. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 7, **dadurch gekennzeichnet, dass** die Ölkomponente ausgewählt ist aus der Gruppe, bestehend aus 2-Ethylhexylisostearat, Octyldodecanol, Isotridecylisononanoat, Isoeicosan, 2-Ethylhexylcocoat, C₁₂₋₁₅- Alkylbenzoat, Capryl-Caprinsäuretriglycerid, Dicaprylylether und deren Mischungen.

11. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 7, **dadurch gekennzeichnet, dass** die Ölkomponente ausgewählt ist aus der Gruppe, bestehend aus Mischungen aus C₁₂₋₁₅-Alkylbenzoat und 2-Ethylhexylisostearat, Mischungen aus C₁₂₋₁₅-Alkylbenzoat und Isotridecylisononanoat und Mischungen aus C₁₂₋₁₅-Alkylbenzoat, 2-Ethylhexylisostearat und Isotridecylisononanoat.

12. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 7, **dadurch gekennzeichnet, dass** die Ölkomponente eine Mischung aus Octamethylcyclotetrasiloxan und Isotridecylisononanoat und Mischungen aus Octamethylcyclotetrasiloxan und 2-Ethylhexylisostearat umfasst.

13. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, juckreiz und -quellung gemäß irgendeinem der vorangehenden Ansprüche 1 bis 12, **dadurch gekennzeichnet, dass** der mindestens eine übliche kosmetische und/oder pharmazeutische Hilfs-, Verarbeitungs- und/oder Zusatzstoff ausgewählt ist aus der Gruppe, bestehend aus Emulgatoren, Coemulgatoren, Stabilisatoren, Dispergatoren, Verdickungsmitteln, Feuchthaltemitteln, Konservierungsstoffen, Duftstoffen, Chemikalien, Geruchsabsorbern, Pigmenten, Farbstoffen, desodorierenden Wirkstoffen, filmbildenden Stoffen, Lichtschutzmitteln, Antioxidantien, Enzymen, Weichmachern, fungistatischen Mitteln, bakteriostatischen Mitteln und Pflegestoffen.

14. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -juckreiz und -quellung gemäß Anspruch 13, **dadurch gekennzeichnet, dass** das Verdickungsmittel ausgewählt ist aus der Gruppe, umfassend Hydroxyethylcellulose, Hydroxypropylcellulose, Polyacrylsäure, Polyvinylpyrrolidon, Polydimethylsiloxan, Polymethylphenylsiloxan, Polyacrylat, Siliciumdioxid und Aluminiumsilikat.

15. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, -Juckreiz und -quellung gemäß Anspruch 14,
**dadurch gekennzeichnet, dass** das Verdickungsmittel bei ölig-alkoholischen Gelen Siliciumdioxid oder ein Aluminiumsilikat ist und bei wässerig-alkoholischen oder alkoholischen Gelen ein Polyacrylat ist.

16. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, juckreiz und -quellung gemäß irgendeinem der vorstehenden Ansprüche 1 bis 15, die 5 bis 30 % Aluminiumchlorohydrat und 2 bis 15 % Aluminiumchlorohydratlactat und/oder -citrat, Glycerin, Sorbitol, Propylenglycol, Ethoxydiglycol, Aloe vera, Konjac-Manane, Hyaluronsäure, Squalan und einem pH-Wert zwischen 4,0 bis 7,0 aufweist.

17. Verwendung der Zusammensetzung mit hautentquellender Wirkung zur Herstellung eines topischen Präparats zur Prophylaxe und/oder Behandlung von Hautekzemen, -rötungen, - trockenheit, -einrissen, -schuppungen, juckreiz und -quellung gemäß irgendeinem der vorangehenden Ansprüche 1 bis 16 in Form von Gel, Lipogel, Creme, Salbe, Lotion, Schaum, mit und ohne Treibmittel oder Druckgas.

## Claims

1. Use of a composition having a skin-shrinking effect for producing a topic preparation for the prophylaxis and/or treatment of skin eczema, skin redness, skin dryness, skin defects, skin scaliness, skin itchiness and skin swelling, wherein the composition has aluminium chlorohydrate and at least one trivalent aluminium salt with an organic ligand, selected from aluminium chlorohydrate lactate and/or aluminium chlorohydrate citrate and/or aluminium chlorohydrate malate, including their alkaline and alkaline earth salts and, as a base, a pharmaceutically acceptable carrier and possibly one or more conventional cosmetic and/or pharmaceutical adjuvants, processing substances and/or additional substances, the composition having a pH value of between 4.0 and 7.0.

2. Use of the composition having a skin-shrinking effect for producing a topic preparation for the prophylaxis and/or treatment of skin eczema, skin redness, skin dryness, skin defects, skin scaliness, skin itchiness and skin swelling according to claim 1, **characterised in that** the composition contains aluminium chlorohydrate in a concentration of 5 to 30 % by wt. and the aluminium salt in a concentration of 2 to 10 % by wt.

3. Use of the composition having a skin-shrinking effect for producing a topic preparation for the prophylaxis and/or treatment of skin eczema, skin redness, skin dryness, skin defects, skin scaliness, skin itchiness and skin swelling according to claim 1 or 2, **characterised in that** the pH value is 5.5.

4. Use of the composition having a skin-shrinking effect for producing a topic preparation for the prophylaxis and/or treatment of skin eczema, skin redness, skin dryness, skin defects, skin scaliness, skin itchiness and skin swelling according to any of the foregoing claims 1 to 3, **characterised in that** the carrier includes at least one liquid base material, at least one pH regulator, and possibly at least one oil component.

5. Use of the composition having a skin-shrinking effect for producing a topic preparation for the prophylaxis and/or treatment of skin eczema, skin redness, skin dryness, skin defects, skin scaliness, skin itchiness and skin swelling according to claim 4, **characterised in that** the liquid base material includes water, alcohols having 2 to 4 carbon atoms or mixtures thereof.

6. Use of the composition having a skin-shrinking effect for producing a topic preparation for the prophylaxis and/or treatment of skin eczema, skin redness, skin dryness, skin defects, skin scaliness, skin itchiness and skin swelling according to claim 4, **characterised in that** the pH regulator includes lactic acid, citric acid, lactic acid salts, citric acid salts or mixtures thereof, the salts more especially including alkaline and alkaline earth salts, or alkali.

7. Use of the composition having a skin-shrinking effect for producing a topic preparation for the prophylaxis and/or treatment of skin eczema, skin redness, skin dryness, skin defects, skin scaliness, skin itchiness and skin swelling according to claim 4, **characterised in that** the oil component includes esters of saturated and/or unsaturated, branched and/or non-branched alcohols with a chain length of 3 to 30 carbon atoms and saturated and/or unsaturated, branched and/or non-branched alkane carboxylic acids with a chain length of 3 to 30 carbon atoms and/or aromatic carboxylic acids, hydrocarbons, hydrocarbon waxes, silicone oils, dialkyl ethers, saturated and/or unsaturated, branched and/or non-branched alcohols and fatty acid triglycerides and mixtures thereof.

8. Use of the composition having a skin-shrinking effect for producing a topic preparation for the prophylaxis and/or treatment of skin eczema, skin redness, skin dryness, skin defects, skin scaliness, skin itchiness and skin swelling according to claim 7, **characterised in that** the oil component is selected from the group of ester oils, comprising isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethyl hexyl palmitate, 2-ethyl hexyl laurate, 2-hexyl decyl stearate, 2-octyl dodecyl palmitate, oleyl erucate, erucyl oleate, erucyl erucate, as well as synthetic, half-synthetic and natural mixtures of such esters, from the group of triglycerin esters of saturated and/or unsaturated, branched and/or non-branched alkane carboxylic acids with a chain length of 8 to 24, preferably 12 to 18 carbon atoms, from the group of synthetic, half-synthetic and natural oils, comprising olive oil, sunflower oil, soya oil, groundnut oil, rapeseed oil, almond oil, palm oil, coconut oil, jojoba oil, mink oil and castor oil, from the group of hydrocarbons and waxes, comprising paraffin oil, squalane and squalene, from the group of silicone oils, comprising octamethyl cyclotetrasiloxane, hexamethyl cyclotrisiloxane, polydimethyl siloxane, poly(methyl phenyl siloxane) and mixtures thereof.

9. Use of the composition having a skin-shrinking effect for producing a topic preparation for the prophylaxis and/or treatment of skin eczema, skin redness, skin dryness, skin defects, skin scaliness, skin itchiness and skin swelling according to claim 7, **characterised in that** the oil component is cetyl palmitate.

10. Use of the composition having a skin-shrinking effect for producing a topic preparation for the prophylaxis and/or treatment of skin eczema, skin redness, skin dryness, skin defects, skin scaliness, skin itchiness and skin swelling according to claim 7, **characterised in that** the oil component is selected from the group comprising 2-ethyl hexyl isostearate, octyl dodecanol, isotridecyl isononanoate, isoeicosane, 2-ethyl hexyl cocoate, C₁₂₋₁₅-alkyl benzoate, caprylic-capric acid triglyceride, dicaprylyl ether and mixtures thereof.

11. Use of the composition having a skin-shrinking effect for producing a topic preparation for the prophylaxis and/or treatment of skin eczema, skin redness, skin dryness, skin defects, skin scaliness, skin itchiness and skin swelling according to claim 7, **characterised in that** the oil component is selected from the group comprising mixtures of C₁₂₋₁₅-alkyl benzoate and 2-ethyl hexyl isostearate, mixtures of C₁₂₋₁₅-alkyl benzoate and isotridecyl isononanoate and mixtures of C₁₂₋₁₅-alkyl benzoate, 2-ethyl hexyl isostearate and isotridecyl isononanoate.

12. Use of the composition having a skin-shrinking effect for producing a topic preparation for the prophylaxis and/or treatment of skin eczema, skin redness, skin dryness, skin defects, skin scaliness, skin itchiness and skin swelling according to claim 7, **characterised in that** the oil component includes a mixture of octamethyl cyclotetrasiloxane and isotridecyl isononanoate and mixtures of octamethyl cyclotetrasiloxane and 2-ethyl hexyl isostearate.

13. Use of the composition having a skin-shrinking effect for producing a topic preparation for the prophylaxis and/or treatment of skin eczema, skin redness, skin dryness, skin defects, skin scaliness, skin itchiness and skin swelling according to any of the preceding claims 1 to 12, **characterised in that** the at least one conventional cosmetic and/or pharmaceutical adjuvant, processing substance and/or additional substance is selected from the group comprising emulsifiers, co-emulsifiers, stabilisers, dispersifiers, thickeners, moisture retainers, preservatives, perfumes, chemicals, odour absorbers, pigments, dyestuffs, deodorising active substances, film-forming substances, light protection agents, antioxidants, enzymes, plasticizers, fungistatic agents, bacteriostatic agents and caring substances.

14. Use of the composition having a skin-shrinking effect for producing a topic preparation for the prophylaxis and/or treatment of skin eczema, skin redness, skin dryness, skin defects, skin scaliness, skin itchiness and skin swelling according to claim 13, **characterised in that** the thickener is selected from the group including hydroxyethyl cellulose, hydroxypropyl cellulose, polyacrylic acid, polyvinyl pyrrolidone, polydimethyl siloxane, polymethyl phenyl siloxane, polyacrylate, silicon dioxide and aluminium silicate.

15. Use of the composition having a skin-shrinking effect for producing a topic preparation for the prophylaxis and/or treatment of skin eczema, skin redness, skin dryness, skin defects, skin scaliness, skin itchiness and skin swelling according to claim 14, **characterised in that** the thickener for oily-alcoholic gels is silicon dioxide or an aluminium silicate and is a polyacrylate for aqueous-alcoholic or alcoholic gels.

16. Use of the composition having a skin-shrinking effect for producing a topic preparation for the prophylaxis and/or treatment of skin eczema, skin redness, skin dryness, skin defects, skin scaliness, skin itchiness and skin swelling according to any of the foregoing claims 1 to 15, which composition has 5 to 30 % aluminium chlorohydrate and 2 to 15 % aluminium chlorohydrate lactate and/or citrate, glycerin, sorbitol, propylene glycol, ethoxydiglycol, aloe vera, konjac-mannan, hyaluronic acid, squalane and a pH value between 4.0 and 7.0.

17. Use of the composition having a skin-shrinking effect for producing a topic preparation for the prophylaxis and/or treatment of skin eczema, skin redness, skin dryness, skin defects, skin scaliness, skin itchiness and skin swelling according to any of the preceding claims 1 to 16 in the form of gel, lipogel, cream, ointment, lotion, foam, with and without propellants or pressure gas.

## Revendications

1. Utilisation d'une composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané, la composition contenant du chlorhydrate d'aluminium et au moins un sel d'aluminium trivalent avec un ligand organique, choisi parmi le chlorhydrolactate d'aluminium et/ou le chlorhydrocitrate d'aluminium et/ou le chlorhydromalate d'aluminium, incluant leurs sels alcalins et alcalino-terreux et contenant comme base un porteur pharmaceutiquement acceptable et éventuellement un ou plusieurs excipients, substances de transformation et/ou additifs cosmétiques et/ou pharmaceutiques habituels, la composition présentant un pH compris entre 4,0 et 7,0.

2. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 1, **caractérisée en ce que** la composition contient du chlorhydrate d'aluminium à une concentration de 5 à 30 % en poids et le sel d'aluminium à une concentration de 2 à 10 % en poids.

3. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 1 ou selon la revendication 2, **caractérisée en ce que** le pH est de 5,5.

4. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon l'une quelconque des revendications 1 à 3, **caractérisée en ce que** le porteur comprend au moins un matériau de base liquide, au moins un régulateur du pH et le cas échéant au moins un composant huileux.

5. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 4, **caractérisée en ce que** le matériau de base liquide comprend de l'eau, un alcool avec 2 à 4 atomes de carbone ou leurs mélanges.

6. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 4, **caractérisée en ce que** le régulateur de pH comprend l'acide lactique, l'acide citrique, des sels d'acide lactique, des sels d'acide citrique ou leurs mélanges, les sels comprenant en particulier des sels alcalins ou alcalino-terreux, ou des alcalis.

7. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 4, **caractérisée en ce que** le composant huileux comprend un ester d'alcools saturés et/ou insaturés, ramifiés ou linéaires, d'une longueur de chaîne de 3 à 30 atomes de carbone et des acides alcane-carboxyliques saturés et/ou insaturés, ramifiés et/ou linéaires d'une longueur de chaîne de 3 à 30 atomes de carbone, et/ou des acides carboniques aromatiques, des hydrocarbures, des cires d'hydrocarbure, de l'huile de silicone, du dialkyléther, des alcools saturés et/ou insaturés, ramifiés et/ou linaires et des triglycérides d'acide gras et leurs mélanges.

8. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 7, **caractérisée en ce que** le composant huileux est choisi dans le groupe comprenant les huiles ester, comprenant l'isopropylmyristate, l'isopropylpalmitate, l'isopropylstéarate, l'isopropyloléate, le n-butylstéarate, le n-hexyllaurate, le n-décyloléate, l'isooctylstéarate, l'isononylstéarate, l'isononylisononanoate, le 2-éthylhexylpalmitate, le 2-éthylhexyllaurate, le 2-hexyldécylstéarate, le 2-octyldodécylpalmitate, l'oléylérucate, l'érucyloléate, l'érucylérucate, ainsi que des mélanges synthétiques, semi-synthétiques et naturels de ces esters, dans le groupe des esters de triglycérine d'acides alcane-carboxyliques saturés et/ou insaturés, ramifiés et/ou linéaires d'une longueur de chaîne de 8 à 24, de préférence de 12 à 18 atomes de carbone, dans le groupe des huiles synthétiques, semi-synthétiques et naturelles comprenant l'huile d'olive, l'huile de tournesol, l'huile de soja, l'huile de noix, l'huile de colza, l'huile d'amande, l'huile de palme, l'huile de noix de coco, l'huile de jojoba, l'huile de vison et l'huile de ricin, dans le groupe des hydrocarbures et des cires d'hydrocarbures, comprenant l'huile de paraffine, le squalane et le squalène, dans le groupe des huiles de silicone, comprenant l'octaméthylcyclotétrasiloxane, l'hexaméthylcyclotrisiloxane, le polydiméthylsiloxane, le poly(méthylphénylsiloxane) et leurs mélanges.

9. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 7, **caractérisée en ce que** le composant huileux est du cétylpalmitate.

10. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 7, **caractérisée en ce que** le composant huileux est choisi dans le groupe comprenant le 2-éthylhexylisostéarate, l'octyldodécanl, l'isotridécylisononanoate, l'isoéicosan, le 2-éthylhexylcocoate, l'alkylbenzoate C₁₂₋₁₅, le triglycéride d'acide caprylique - caprique, le dicaprylyléther et leurs mélanges.

11. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 7, **caractérisée en ce que** le composant huileux est choisi dans le groupe comprenant des mélanges d'alkylbenzoate C₁₂₋₁₅ et de 2-éthylhexylisostéarate, des mélanges d'alkylbenzoate C₁₂₋₁₅ et d'isotridécylisononanoate et des mélanges d'alkylbenzoate C₁₂-₁₅, de 2-éthylhexylisostéarate et d'isotridécylisononanoate.

12. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 7, **caractérisée en ce que** le composant huileux comprend un mélange d'octaméthylcyclotétrasiloxane et d'isotridécylisononanoate et des mélanges d'octaméthylcyclotétrasiloxane et de 2-éthylhexylisostéarate.

13. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon l'une quelconque des revendications précédentes 1 à 12, **caractérisée en ce que** le au moins un excipient, substance de transformation et/ou additif cosmétique et/ou pharmaceutique habituel est choisi dans le groupe comprenant des émulsifiants, des co-émulsifiants, des stabilisants, des dispersants, des épaississants, des hydratants, des conservateurs, des parfums, des produits chimiques, des absorbeurs d'odeur, des pigments, des colorants, des substances désodorisantes, des substances filmogènes, des agents de protection contre la lumière, des arti-oxydants, des enzymes, des assouplissants, des antifongiques, des bactériostatiques et des substances de soin.

14. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 13, **caractérisée en ce que** l'épaississant est choisi dans le groupe comprenant l'hydroxyéthylcellulose, l'hydroxypropylcellulose, l'acide polyacrylique, la polyvinylpyrrolidone, le polydiméthylsiloxane, le polyméthylphénylsiloxane, le polyacrylate, le dioxyde de silicium et le silicate d'aluminium.

15. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon la revendication 14, **caractérisée en ce que** l'épaississant est du dioxyde de silicium ou du silicate d'aluminium pour les gels huileux-alcooliques et un polyacrylate pour les gels aqueux - alcooliques ou alcooliques.

16. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon l'une quelconque des revendications précédentes 1 à 15, qui contient 5 à 30 % de chlorhydrate d'aluminium et 2 à 15% de chlorhydrolactate et/ou de chlorhydrocitatre d'aluminium, de la glycérine, du sorbitol, du propylène glycol, de l'éthoxyglycol, de l'aloe vera, du Konjac - Mannane, de l'acide hyaluronique, du Squalane, et présente un pH entre 4,0 et 7,0.

17. Utilisation de la composition ayant un effet de dégonflement de la peau pour la fabrication d'une préparation topique pour la prophylaxie et/ou le traitement de l'eczéma cutané, des rougeurs cutanées, de la sécheresse cutanée, des écorchures, de la desquamation cutanée, des démangeaisons cutanées et du gonflement cutané selon l'une quelconque des revendications précédentes 1 à 16 sous la forme d'un gel, d'un lipogel, d'une crème, d'un onguent, d'une lotion, d'une mousse, avec ou sans agent propulseur ou gaz comprimé.
